# EUROPEAN PATENT SPECIFICATION

(11) **EP 1 450 788 B1**
(45) Date of publication and mention of the grant of the patent: **21.09.2005**
(21) Application number: 01274892.7
(22) Date of filing: 04.12.2001
(51) Int. Cl.: A61K 31/37, A61K 9/20, A61K 9/48, A61P 7/10

(54) **USE OF COUMARIN OR ITS HYDROXY DERIVATIVE FOR THE TREATMENT OF MEDIUM OR SEVERE VASCULAR AND/OR LYMPHATIC EDEMA**
VERWENDUNG VON CUMARIN ODER SEINES HYDROXY-DERIVATS ZUR BEHANDLUNG VON MITTELSCHWEREM ODER SCHWEREM GEFÄSS- UND/ODER LYMPHÖDEM
UTILISATION DE COUMARINE OU DE DERIVES HYDROXYLES DE COUMARINE POUR LE TRAITEMENT D'UN OEDEME LYMPHATIQUE ET/OU VEINEUX MOYEN OU GRAVE

(43) Date of publication of application: 01.09.2004
(73) Proprietor: MAC PHARMA S.A.S., 31100 Treviso (IT)
(72) Inventor: CHIMENTI, Massimo, I-30033 Noale (IT); FRATTER, Andrea, I-31022 Preganziol (IT)
(74) Representative: Cattaneo, Elisabetta
(86) International application number: PCT/IT2001/000613
(87) International publication number: WO 2003/047566

(56) References cited:
- EP-A- 0 185 319
- EP-A- 0 668 074
- WO-A-85/03865
- WO-A-99/21550
- DE-A- 3 715 990
- US-A- 4 616 033
- BURGOS A ET AL: "Comparative study of the clinical efficacy of two different coumarin dosages in the management of arm lymphedema after treatment for breast cancer." LYMPHOLOGY, vol. 32, no. 1, March 1999 (1999-03), pages 3-10, XP008005902 ISSN: 0024-7766
- HOPF, G. ET AL: "Experimental studies on the effect of coumarin on postoperative extremity edema in the dog" ARZNEIM.-FORSCH. (1971), 21(6), 854-5 , XP001074082
- PILLER N P: "Benzopyrone treatment of mild thermal oedema: determination of the most effective doses." ARZNEIMITTEL-FORSCHUNG. GERMANY, WEST 1977, vol. 27, no. 6, 1977, pages 1138-1141, XP001074079 ISSN: 0004-4172
- L. CLODIUS AND N.B. PILLER: "Conservative Therapy for Postmastectomy Lymphedema" CHIR. PLASTICA, vol. 4, 1978, pages 193-202, XP008006092
- CASLEY-SMITH J R: "BENZO-PYRONES IN THE TREATMENT OF LYMPHOEDEMA" INTERNATIONAL ANGIOLOGY, TORINO, IT, vol. 1, no. 18, March 1999 (1999-03), pages 31-41, XP008005903 ISSN: 0392-9590
- "Dorland's Illustrated Medical Dictionary, 24Th Ed." 1965, SAUNDERS CY. , PHILADELPHIA, U.S. * page 926 *
- MUTSCHLER: "Mutschler Arzneimittelwirkungen, Lehrbuch der Pharmakologie und Toxicologie" February 2001 (2001-02), WISSENSCHAFTLICHE VERLAGSGESELLSCHAFT MBH STUTTGART , STUTTGART * pages 11-15 *
- retrieved from HTTP://WWW.BIAM2.ORG/WWW/SPE1149.HTM

## Description

The present invention concerns the use of benzo-pyrone derivatives for the treatment of vascular and/or lymphatic edema of middle and high amplitude. Particularly the invention concerns the treatment of vascular and/or lymphatic edema of middle and high amplitude through the use of coumarin (5,6-benzopyran-2-one) or its hydroxy derivative, 7-hydroxycoumarin, also named umbelliferone.

The therapy of lymphoedema of middle and high amplitude nowadays makes use of a pharmaceutical therapy based on some diuretic, anticoagulants, flebotonic drugs, antibiotics for the infective complications, but only in order to complete the surgical treatment, elasto-compressive treatment, manipulative treatment and in any case they are non sufficient to eliminate the aetiology of such anatomic, physio-pathological manifestations.

As a matter of fact in order to apply an effective therapy it is necessary to use a synergism of more approaches such as pharmaceutical, elasto-compressive and manual lympho-drainage treatments.

It is well known to use a lot of drugs able to be effective at the vein-lymphatic level in order to treat lymphoedema and hyperproteic angioedema of the limbs.

Among these drugs attention was paid to benzopyrones derivative, and specifically to coumarin. It is known that coumarin acts with a receptor pharmaco-dynamic mechanism at immuno-competent cell level of white series (monocytes, macrophages, and with a less affect on NK), enhancing the migration to the interested part of the edema of such cells (interleukin-like effect) and the production from the same cells of lytic enzymes, which are able to cleave the proteins which are present in the plasma and are the reason of the high oncotic pressure being responsible of the edema.

The use of coumarin at very high doses, which in very critical cases correspond to 400-800 mg/day, for the treatment of lymphoedema, is well known. When the amounts of coumarin are so high some cases of even serious hepatic toxicity are described. Some studies pointed out the possible toxicity of the coumarin at the hepatic tissue level after a repeated systemic administration (especially orally) of variable doses. According to such studies it seems that oral doses of coumarin of about 200 mg/day are associated to high concentrations of some cytosolytic hepatocytic enzymes in the plasma, which are biochemical markers of the hepatic damage. Other studies supposed that the possible tissue toxicity after a chronic acute administration of coumarin should be ascribed to idiosyncrasy and hence to genetic and immunologic factors rather than the formation of toxic compounds due to the action of the said enzymes.

Specific amounts of coumarin were tempted in "Comparative Study of the clinic efficacy of two different coumarin dosages in the management of arm lymphoedema after treatment of breast cancer [Lymphology, 32(1), 3-10, 1999]. Specifically the clinical efficacy of coumarin 90 mg/day and 135 mg/day were compared in patients with lymphoedema of the upper limbs secondary to surgery and irradiation for treatment of the breast cancer. No difference was found between the apparent benefits of the two coumarin dosages. Data obtained for all patients were also considered for the toxicity and a low hepatotoxicity was observed (column 9). Therefore it is evident the difficulty of reaching a balance between the efficacy needs and the hepatotoxity needs when coumarin is administered.

Regardless of the mechanism causing the tissue toxicity, the toxicity cases made coumarin to be abandoned in the lymphoedema therapy.

Subsequently some experts tried a safer use of coumarin starting from melilotus officinalis extracts, corresponding to a 5-25 mg/capsule of coumarin as melilotus officinalis extract. On one hand the clinic titration allowed a use of coumarin to be safer, on the other hand it resulted in being clinically insignificant in the case of vascular and lymphatic edema of middle and high amplitude.

An example of the use of Melilotus officinalis titrated in coumarin in a treatment of postoperative edemas is described in Hopf, G. et al "Experimental Studies on the effect of coumarin on postoperative extremity edema in the dog" Arxneim. Forsch. (1971), 21(6), 854-5.

In order to avoid the hepatotoxicity of coumarin or a derivative thereof a different route of administration other than oral route is suggested in European patent application n. EP0668074, wherein the transdermal administration of coumarin, or its derivative, i.e. 7- hydroxy derivative, is suggested. The application of such transdermal systems allows an high concentration of benzopyrons in the target tissue with a contemporaneous low decrease of the toxycological effect to be obtained. Through this route the haematic levels of coumarin were 10 fold higher than those through the oral route, while for 7-hydroxy-coumarin the transdermic route has revealed a decrease of the haematic levels. As a matter of facts, in view of the toxycological problem as alternative derivative of coumarin, 7-hydroxy coumarin or umbelliferone was also proposed.

Umbelliferone is the metabolic intermediate of coumarin and it is the most important metabolic intermediate in vivo in the human species. Such 7-hydroxy derivative was resulted from some studies absolutely not involved in the generation of the above described idiosyncratic hepatopathy which is generated by the formation in vivo of epoxydate derivatives in position 3 and 4 of alfa-pyronic ring. As a matter of fact the *in vivo* coumarin metabolization which provides for the formation of 7-hydroxy derivative or umbelliferone, facilitates the formation of 7-glycuronides, which are easily eliminable by the renal emunctory action.

The object of the present invention is hence to provide a therapy of treatment of lymphoedema of middle and high amplitude while contemporaneously avoiding the toxicological problem associated to coumarin.

The above indicated objects are achieved through the use of coumarin or its hydroxy derivative for the preparation of a drug for the treatment of vascular and/or lymphatic edema of middle and high amplitude, wherein the amount of sublingually in a micronised form coumarin or its hydroxy derivative is administered as recited in claim 1. Advantages and characteristics are recited in the dependent claims.

In order to treat these kinds of vascular and/or lymphatic edema the coumarin or its hydroxy derivative is preferably administered in amount of 35-55 mg. More Preferably the amount the coumarin or its hydroxy derivative is of 45 mg pro-unit.

In the attempt of making minimal the risk of hepatotoxicity therefore a micronized pharmaceutical form for sublingual administration has been envisaged, using as administration route the sublingual capillary network which, not only guarantees a extremely rapid systemic absorption, being the sublingual mucosa extremely vascularized, but also allows to bypass the portal circulation avoiding a spread hepatic passage. Particularly coumarin or its hydroxy derivative is subjected to a process of micronization in order to reach a granule size of 2 µm diameter or less. Therefore when the term "micronized" is used, it means a granule size of 2 µm diameter or less.

The micronized form for sublingual administration can be in the form of sublingual tablet.

Coumarin or its 7-hydroxy derivative in suitable amounts can then be added with suitable excipients, which are well known to the man skilled in the art of the preparation of drug administration pharmaceutical forms. Suitable excipients for the sublingual preparation are e.g. microcristalline cellulose, corn starch, magnesium stearate, sodium metabisolphite, silica gel; for the transdermal administration hydroxyethylcellulose, carboxyethylcellulose, PEG, alone or with other balsamic agents or local anaesthetic agents such as menthol, benzocaine, procaine, lidocaine, tetracaine and the like. Preferable solvents for dissolution of coumarin are ethanol or other lyposoluble organic solvent as chloroform, ethyl ether, toluene, oils and other alkalis such as alkaline hydrates. Preferable solvents for the solution of umbelliferone are ethanol, dichloromethane, chloroform and any other lyposoluble aliphatic or aromatic solvents.

When coumarin or umbelliferone are in solution a local anaesthetic agent or balsamic agent can be added (e.g. benzocaine and menthol) in order to avoid the possible local irritation. According to the present invention the amount of excipients will not be above the range of 65-85 mg, being coumarin or its 7-hydroxy derivative amount comprised between 35 and 55 mg pro drug unit. More preferably the amount of excipients will not be above 75 mg pro unit, the coumarin amount being 45 mg pro unit. According to the present invention the tablets will have preferably sizes of about 8 mm diameter and of 2-3 mm thickness so as to assure the easiest administration and hence patient compliance, contemporaneously satisfying an effective sublingual absorption.

Preferably the treatment protocol comprises the administration of the claimed coumarin or its hydroxy derivative amount once a day, preferably during the daytime hours in order to avoid the excessive night diuresis associated to coumarin compounds.

Some illustrative embodiments of the medicament as sublingual tablet according to the invention follow by way of example.

### Preparation of a sublingual tablet containing coumarin

450 g of coumarin as polymorphous crystals were subjected to a process of making a powder through a steel ball mill, obtaining a powder consisting of particles of size of 2 µm. The sieving and size checking steps of the obtained particles were carried out through microscope.

The so obtained powder was mixed with 12 g of micronized talc, 60 g of microcrystalline cellulose powder, 350 g of lactose powder through a screw mixer. The so obtained mixture was subjected to a wet fluid bed granulation process by using the minumum amount of sucrose solution, and the obtained granulate was then kneaded, sieved and dried. The size check of the granules was then carried out.

Granules were then pressed and tablets of 8 mm diameter and weight of 120 mg were obtained. They were subjected to technological and biopharmaceutical examination as from official Pharmacopoeia. The composition of each tablet was as follows:
Coumarin: 37,5%
Microcrystalline cellulose: 6,6% by weight
corn starch: 25% by weight
Magnesium stearate: 8,3% by weight
Sodium metabisulphite: 0,3% by weight
Silica gel: 4,2% by weight
Saturated sucrose solution (for coating): q.s. to 100%

### Preparation of a sublingual tablet containing 7-hydroxycoumarin

450 g of 7-hydroxycoumarin as polymorphous crystals were subjected to a process of making a powder through a steel ball mill; obtaining a powder composed of particles of size of about 2 µm. The sieving and size checking steps of the obtained particles were carried out through microscope.

The so obtained powder was mixed with 12 g of micronized talc, 60g of microcrystalline cellulose powder, 350g of lactose powder through a screw mixer. The so obtained mixture was subjected to a granulation wet fluid bed process by using the minimum amount of sucrose solution, and the obtained granulate was then kneaded, sieved and dried. The size check of the granules was then carried out.

Granules were then pressed and tablets of 8 mm diameter and weight of 120 mg were obtained. They are subjected to technological and biopharmaceutical examination as from official Pharmacopoeia. The composition of each tablet was as follows:
7-hydroxycoumarin: 37,5%
Microcrystalline cellulose: 6,6% by weight
corn starch: 25% by weight
Magnesium stearate: 8,3% by weight
Sodium metabisulphite: 0,3% by weight
Silica gel: 4,2% by weight
Saturated sucrose solution (for coating): q.s. to 100%

## Claims

1. Use of micronized coumarin or 7-hydroxycoumarin with a granule size of 2 µm or less for the preparation of a medicament for the treatment of lymphatic and/or vascular edema of middle and high amplitude by sublingual administration.

2. Use according to claim 1 wherein the amount of coumarin or 7-hydroxycoumarin in the medicament is between 35 and 55 mg pro drug unit.

3. Use according to claim 2 wherein the amount of coumarin or 7-hydroxycoumarin in the medicament is 45 mg pro drug unit.

4. Use according to anyone of the preceding claims wherein the excipients are between 65 and 85 mg pro drug unit.

5. Use according to anyone of the preceding claims wherein the vascular and/or lymphatic edema is associated to flebolymphostatic ulcer and/or dermatolymphangioadhenite.

## Patentansprüche

1. Verwendung von feinstzerkleinertem Cumarin oder 7-Hydroxycumarin mit einer Korngröße von 2 µm oder weniger für die Herstellung eines Medikamentes zur Behandlung eines mittelschweren oder schweren Lymph- uhd/oder Gefäßödems durch sublinguale Verabreichung.

2. Verwendung nach Anspruch 1, wobei die Menge von Cumarin oder 7-Hydroxycumarin im Medikament 35 bis 55 mg pro Medikamenten-Einheit beträgt.

3. Verwendung nach Anspruch 2, wobei die Menge von Cumarin oder 7-Hydroxycumarin im Medikament 45 mg pro Medikamenten-Einheit beträgt.

4. Verwendung nach einem der vorstehenden Ansprüche, wobei die Arzneimittelträger mit 65 bis 85 mg pro Medikamenten-Einheit vorliegen.

5. Verwendung nach einem der vorstehenden Ansprüche, wobei das Gefäß- und/oder Lymphödem mit einem flebolymphostatischen Geschwür und/oder Dermatolymphagioadhenit verbunden ist.

## Revendications

1. Utilisation de coumarine ou de 7-hydroxy-coumarine micronisée ayant une taille de granule de 2 µm ou moins, pour la préparation d'un médicament pour le traitement d'un oedème lymphatique et/ou vasculaire d'importance moyenne et élevée, par administration sublinguale.

2. Utilisation selon la revendication 1, dans laquelle la quantité de coumarine ou de 7-hydroxy-coumarine dans le médicament est entre 35 et 55 mg par unité médicamenteuse.

3. Utilisation selon la revendication 2, dans laquelle la quantité de coumarine ou de 7-hydroxy-coumarine dans le médicament est de 45 mg par unité médicamenteuse.

4. Utilisation selon l'une quelconque des revendications précédentes, dans laquelle les excipients sont entre 65 et 85 mg par unité médicamenteuse.

5. Utilisation selon l'une quelconque des revendications précédentes, dans laquelle l'oedème vasculaire et/ou lymphatique est associé à un ulcère phlébolymphostatique et/ou une dermatolymphangioadénite.
